# EUROPEAN PATENT SPECIFICATION

(11) **EP 3 690 390 B1**
(45) Date of publication and mention of the grant of the patent: **01.01.2025**
(21) Application number: 20155081.1
(22) Date of filing: 03.02.2020
(51) Int. Cl.: G01B 11/00, G01N 1/22, G01N 33/00, G01N 29/02, G01N 29/22, G01N 29/265, G05D 1/00

(54) **ELECTRONIC NOSE POSITIONING DEVICE AND POSITIONING METHOD**
VORRICHTUNG UND VERFAHREN ZUR POSITIONIERUNG EINER ELEKTRONISCHEN NASE
DISPOSITIF ET PROCÉDÉ DE POSITIONNEMENT DE NEZ ÉLECTRONIQUE

(30) Priority: 03.02.2019 CN 201910108228
(43) Date of publication of application: 05.08.2020
(73) Proprietor: Nuctech Company Limited, TongFang Building, Shuangqinglu, Haidian District Beijing 100084 (CN); Nuctech (Beijing) Company Limited, Beijing 101500 (CN); Tsinghua University, Haidian District, Beijing 100084 (CN)
(72) Inventor: QIAO, Guina, BEIJING, 100084 (CN); WANG, Yongming, BEIJING, 100084 (CN); RAN, Zhansen, BEIJING, 100084 (CN); XU, Yanwei, BEIJING, 100084 (CN); YU, Weifeng, BEIJING, 100084 (CN); YANG, Xuejing, BEIJING, 100084 (CN); HU, Yu, BEIJING, 100084 (CN); ZONG, Chunguang, BEIJING, 100084 (CN); SUN, Shangmin, BEIJING, 100084 (CN)
(74) Representative: Meissner Bolte Partnerschaft mbB

(56) References cited:
- WO-A1-2017/206941
- DE-A1- 19 710 913
- US-A1- 2006 221 877
- US-A1- 2017 184 392
- US-A1- 2017 369 168
- LILIENTHAL A ET AL: "Creating gas concentration gridmaps with a mobile robot", PROCEEDINGS OF THE 2003 IEEE/RSJ INTERNATIONAL CONFERENCE ON INTELLIGENT ROBOTS AND SYSTEMS. (IROS 2003). LAS VEGAS, NV, OCT. 27 - 31, 2003; [IEEE/RSJ INTERNATIONAL CONFERENCE ON INTELLIGENT ROBOTS AND SYSTEMS], NEW YORK, NY : IEEE, US, vol. 1, 27 October 2003 (2003-10-27), pages 118 - 123, XP010672326, ISBN: 978-0-7803-7860-5, DOI: 10.1109/IROS.2003.1250615
- POBKRUT THEERAPAT ET AL: "Soil sensing survey robots based on electronic nose", 2013 13TH INTERNATIONAL CONFERENCE ON CONTROL, AUTOMATION AND SYSTEMS (ICCAS 2013), IEEE, 22 October 2014 (2014-10-22), pages 1604 - 1609, XP032705887, ISSN: 2093-7121, [retrieved on 20141216], DOI: 10.1109/ICCAS.2014.6987829

## Description

### TECHNICAL FIELD

The present disclosure relates to the field of detection, and in particular, to an electronic nose positioning device and positioning method.

### BACKGROUND

The electronic nose, which is also referred to as an odor scanner, is a detection instrument which may be applied to fields such as environmental monitoring (such as monitoring toxic and harmful gases), product quality detection (such as quality monitoring of animal and plant products), medical diagnosis, and explosive detection (such as volatile hazard detection). In the related art, the portable electronic nose may be manually operated by a person, but such manner has relatively limited operational scenes.

For odor inspection of enclosed devices such as containers, it is necessary to perform an inhalation inspection by means of an electronic nose after the container is opened. In the case where the container is not opened, it is necessary to force an electronic nose sampler into the door of the container to perform an inhalation inspection. Therefore, the related art has an inspection process which is relatively complicated and consumes a relatively long time, and the sampler is easily damaged during the inspection process.

There have been several patent documents or journal articles issued that cover variations of the electronic nose positioning device and positioning method. For example, the German patent DE19710913A1 discloses a system to test foreign gas in and remove container. The system includes a gas analyzer and a gas removal system. Containers, such as bottles, standing on a conveyor unit are passed under the gas removal system, to take the gas leaving the containers. A measuring system is provided to determine the individual height of the containers upstream from the gas removal system. A height adjusting unit is provided to lower or raise the gas removal system. A control unit controls the height adjusting unit, according to a signal given by the measuring system. The control unit acts such that the distance between the individual containers and the gas removal system is maintained at a constant predetermined value.

Likewise, the conference paper "Creating gas concentration gridmaps with a mobile robot" in PROCEEDINGS OF THE 2003 IEEE/RSJ INTERNATIONAL CONFERENCE ON INTELLIGENT ROBOTS AND SYSTEMS discloses a mobile robot for creating gas concentration gridmaps. The location estimates required for map building were obtained by the external, vision-based absolute positioning system W-CAPS. The experiments were performed with a Koala mobile robot equipped with the Mark III mobile nose, comprising 6 tin oxide sensors manufactured by Figaro. To record the position of the robot the vision-based absolute positioning system W-CAPS was applied, which tracks a distinctly colored object mounted on top of the robot.

The conference paper "Soil sensing survey robots based on electronic nose" in 2013 13TH INTERNATIONAL CONFERENCE ON CONTROL, AUTOMATION AND SYSTEMS (ICCAS 2013), IEEE discloses a soil sensing survey robot. The e-nose system equipped on this robot was operated under real situation at four location, namely, inside a floor room, lawn, dry ground, and vineyard row, to identify the different characteristics of each place. The e-nose robot moved slowly along the selection path with a velocity of 0.1 meter/second, while fan number 1 and 2 were flowing out to make sure that the most of air flow was from the specific ground. The navigation system of the robot is based on the APM rover Mission planer. Two ultrasonic distance-measuring sensors, gyromagnetic, and accelerometer were used to check and avoid the obstacle in the path of robot movement with real time vision from live radio camera sent to the computer using 5.4 GHz radio frequencies.

U.S. Patent Application Publication No. 20170369168 discloses an unmanned aerial vehicle for scent analysis. The unmanned aerial vehicle includes a sensor array to sample ambient air in a location to detect an odor, a pattern recognition unit coupled to a database to identify the odor, wherein the odor is compared to identification information stored in the database, and a mapping unit to estimate an assessment value associated with the odor and to generate an instruction to one or more components of the unmanned aerial vehicle to perform a function when the assessment value exceeds a predetermined threshold value, wherein the function includes activating one or more feedback outputs on the unmanned aerial vehicle.

U.S. Patent Application Publication No. 20170184392 discloses a vehicle guidance system, a method for orientating a vehicle, and an inspection vehicle. The vehicle guidance system includes at least two separate reference devices; a laser scanner device, configured to emit a laser beam signals and scan a sectorial region with the laser beam so as to measure a distance of a straight connection line for connecting the laser scanner device to any of the at least two separate reference devices, and an angle between the corresponding straight connection line and a vehicle body of the vehicle, or an angle between the straight connection lines; a processor, configured to process and store data, and to determine whether the orientation of the vehicle body in real time is deviating from an initial orientation of the vehicle body upon the system starts to operate or not, in accordance with the sensed results by the laser scanner device.

### SUMMARY

In view of this, the embodiments of the present disclosure provide an electronic nose positioning device and positioning method, which can simplify the inspection process of the electronic nose.

In one aspect of the present disclosure, an electronic nose positioning device is provided. The electronic nose positioning device includes: a carrier; an electronic nose disposed on the carrier; an adjusting mechanism configured to adjust the electronic nose to an odor sampling part of an object to be detected; and a laser sensing unit disposed on the carrier, the electronic nose, or the adjusting mechanism, and configured to scan the object to be detected, so as to determine a position of an odor sampling part of the object to be detected, wherein the laser sensing unit includes at least one area laser sensor, and the at least one area laser sensor includes: a first area laser sensor, whose scanning surface is a vertical scanning surface, configured to obtain side profile information of the object to be detected; and a second area laser sensor, whose scanning surface is a horizontal scanning surface, configured to obtain position and motion information of the object to be detected relative to the electronic nose; wherein the electronic nose positioning device further comprises: a controller configured to receive scan data of the first area laser sensor and the second area laser sensor and establish a side profile image of the object to be detected according to the scan data; wherein the controller is configured to calculate a position of the odor sampling part in the side profile image to determine a horizontal distance and a vertical distance of the odor sampling part relative to the object to be detected, and to cause the adjusting mechanism to adjust the electronic nose to the odor sampling part.

In some embodiments, the carrier is movable.

In some embodiments, the electronic nose includes a host and a sampler, the adjusting mechanism includes: a robot arm disposed on the carrier or the host, and the sampler is disposed at a distal end of the robot arm.

In some embodiments, the carrier is movable, the electronic nose includes a host and a sampler, and the adjusting mechanism includes: a robot arm disposed on the carrier or the host, and the sampler is disposed at a distal end of the robot arm; wherein the controller is configured to calculate a position of the odor sampling part of the object to be detected in the side profile image according to the scan data and cause the carrier to move to a position which a motion range of the distal end of the robot arm is capable of covering the odor sampling part.

In some embodiments, the second area laser sensor is located on one side of the object to be detected adjacent to the first area laser sensor or located on one side of the object to be detected away from the first area laser sensor.

In some embodiments, the object to be detected includes a container, wherein the adjusting mechanism is configured to adjust the electronic nose to a vent hole of a container, the laser sensing unit is configured to scan the container, so as to determine a position of the vent hole of the container.

In one aspect of the present disclosure, an electronic nose positioning method is provided. The electronic nose positioning method includes the steps of: scanning an object to be detected by a laser sensing unit; determining a position of an odor sampling part of the object to be detected according to scan data of the laser sensing unit; and adjusting an electronic nose to the odor sampling part of the object to be detected by an adjusting mechanism, wherein the laser sensing unit includes a first area laser sensor whose scanning surface is a vertical scanning surface and a second area laser sensor whose scanning surface is a horizontal scanning surface, and the step of scanning the object to be detected by the laser sensing unit includes: obtaining a side profile information of the object to be detected by the first area laser sensor; and obtaining position and motion information of the object to be detected relative to the electronic nose by the second area laser sensor; wherein the step of determining a position of an odor sampling part of the object to be detected comprises: establishing a side profile image of the object to be detected according to the scan data of the first area laser sensor and the second area laser sensor; calculating a position of the odor sampling part in the side profile image to determine a horizontal distance and a vertical distance of the odor sampling part relative to the object to be detected; and causing the adjusting mechanism to adjust the electronic nose to the odor sampling part.

In some embodiments, the electronic nose includes a host and a sampler, the host is disposed on a movable carrier, the adjusting mechanism includes a robot arm disposed on the carrier or the host, and the sampler is disposed at a distal end of the robot arm; the step of adjusting an electronic nose includes: causing the carrier to move to a position which a motion range of the distal end of the robot arm is capable of covering the odor sampling part according to a position of the odor sampling part on the side profile image; determining a target position of the robot arm by transformation of a coordinate system according to the position of the odor sampling part in the side profile image; and causing the distal end of the robot arm to move to the target position according to the target position, so that a sampling range of the sampler covers the odor sampling part.

In some embodiments, the object to be detected includes a container, wherein the odor sampling part is a vent hole of the container.

Therefore, according to the embodiments of the present disclosure, the position of the odor sampling part of the object to be detected is determined by scanning the object to be detected through a laser sensing unit, and the electronic nose is adjusted to the odor sampling part by an adjusting mechanism, so that the electronic nose can perform odor sampling at the odor sampling part, thereby meeting the relevant inspection requirements. The scanning of the laser sensing unit and adjustment of the adjusting mechanism can effectively simplify the inspection process of the electronic nose, reduce the time consumed by the inspection process, and improve the inspection efficiency.

### BRIEF DESCRIPTION OF THE DRAWINGS

The accompanying drawings, which constitute part of this specification, illustrate exemplary embodiments of the present disclosure and, together with this specification, serve to explain the principles of the present disclosure.

The present disclosure may be more clearly understood from the following detailed description with reference to the accompanying drawings, in which:
Fig. 1 is a schematic structural view according to some embodiments of the electronic nose positioning device of the present disclosure;
Fig. 2 is a schematic structural view according to other embodiments of the electronic nose positioning device of the present disclosure;
Fig. 3 is a schematic view of a mounting structure of an electronic nose and a robot arm according to some embodiments of the electronic nose positioning device of the present disclosure;
Fig. 4 is a schematic view of scanning an object to be detected by a laser sensing unit according to some embodiments of the electronic nose positioning device of the present disclosure;
Fig. 5 is a schematic view of scanning an object to be detected by a laser sensing unit according to other embodiments of the electronic nose positioning device of the present disclosure;
Fig. 6 is a schematic flow chart according to some embodiments of the electronic nose positioning method of the present disclosure;
Fig. 7 is a schematic flow chart of scanning an object to be detected according to some embodiments of the electronic nose positioning method of the present disclosure;
Fig. 8 is a schematic flow chart of determining an odor sampling part according to some embodiments of the electronic nose positioning method of the present disclosure;
Fig. 9 is a schematic flow chart of adjusting a sampling position of an electronic nose according to some embodiments of the electronic nose positioning method of the present disclosure.

It should be understood that the dimensions of the various parts shown in the accompanying drawings are not drawn according to the actual scale. In addition, the same or similar reference signs are used to denote the same or similar components.

### DETAILED DESCRIPTION

Various exemplary embodiments of the present disclosure will now be described in detail with reference to the accompanying drawings. The description of the exemplary embodiments is merely illustrative and is in no way intended as a limitation to the present disclosure and application or use thereof. The present disclosure may be implemented in many different forms, which are not limited to the embodiments described herein. These embodiments are provided to make the present disclosure thorough and complete, and fully convey the scope of the present disclosure to those skilled in the art. It should be noted that, unless otherwise specified, the relative arrangements of the components and steps expounded in these embodiments should be construed as merely illustrative, rather than as a delimitation.

The words "first", "second", and similar words used in the present disclosure do not denote any order, quantity or importance, but merely serve to distinguish different parts. Such similar words as "including" or "containing" mean that the element preceding the word encompasses the elements enumerated after the word, and does not exclude the possibility of encompassing other elements as well. The terms "up", "down", "left", "right", or the like are used only to represent a relative positional relationship, and the relative positional relationship may be changed correspondingly if the absolute position of the described object changes.

In the present disclosure, when it is described that a particular device is located between the first device and the second device, there may be an intermediate device between the particular device and the first device or the second device, and alternatively, there may be no intermediate device. When it is described that a particular device is connected to other devices, the particular device may be directly connected to said other devices without an intermediate device, and alternatively, may not be directly connected to said other devices but with an intermediate device.

All the terms (including technical and scientific terms) used in the present disclosure have the same meanings as understood by those skilled in the art of the present disclosure unless otherwise defined. It should also be understood that terms as defined in general dictionaries, unless explicitly defined herein, should be interpreted as having meanings that are consistent with their meanings in the context of the relevant art, and not to be interpreted in an idealized or extremely formalized sense.

Techniques, methods, and apparatus known to those of ordinary skill in the relevant art may not be discussed in detail.

According to one aspect of the present disclosure, an electronic nose positioning device is provided. The electronic nose positioning device include: a carrier; an electronic nose disposed on the carrier; an adjusting mechanism configured to adjust the electronic nose to an odor sampling part of an object to be detected; and a laser sensing unit disposed on the carrier, the electronic nose, or the adjusting mechanism, and configured to scan the object to be detected, so as to determine a position of an odor sampling part of the object to be detected.

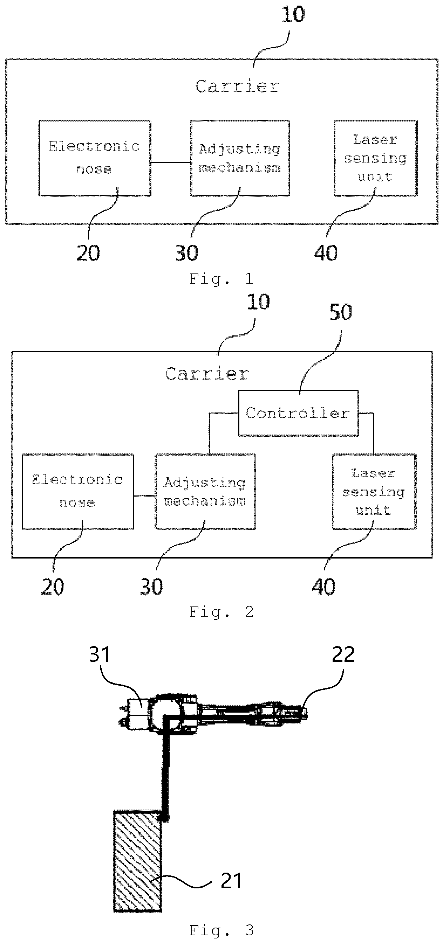

Referring to Fig. 1, the electronic nose positioning device includes: a carrier 10, an electronic nose 20, an adjusting mechanism 30, and a laser sensing unit 40. The carrier 10 may carry various functional devices in the electronic nose positioning device. In some embodiments, the carrier 10 is movable. For example, a running gear is provided at the bottom of the carrier 10 so that it can move on the ground or the surface of a platform, and can implement transition of the device, dynamic scanning of the laser sensor unit 40 in motion, and aid to adjust the electronic nose 20 to an odor sampling part 61 of an object 60 to be detected. In other embodiments, the carrier 10 can be fixed. The carrier 10 can also be a frame adjusted along a track, such as a gantry frame.

The electronic nose 20 is disposed on the carrier 10. Referring to Fig. 3, in some embodiments, the electronic nose 20 can include a host 21 and a sampler 22. The host 21 can be fixed on the carrier 10. In other embodiments, it can also be integrated with the carrier 10. That is, the host 21 also serves as a carrier to carry other functional devices, and a moving gear can be correspondingly provided at the bottom of the host 21.

The adjusting mechanism 30 can adjust electronic nose 20 to an odor sampling part 61 of an object 60 to be detected. Referring to Fig. 3, in some embodiments, the adjusting mechanism 30 can include a robot arm 31. The robot arm 31 can be disposed on the carrier 10 or the host 21, and the sampler 22 is disposed at a distal end of the robot arm 31. The robot 31 moves in multiple degrees of freedom so that the spatial position and sampling direction of the sampler 22 can be adjusted within a wide range. In other embodiments, the adjusting mechanism 30 can also be in other forms. For example, the adjusting mechanism 30 includes a lifting mechanism and a horizontal feeding mechanism to adjust the spatial position of the sampler 22.

The laser sensing unit 40 can be disposed on the carrier 10, the electronic nose 20, or the adjusting mechanism 30. In order to improve scanning efficiency, the laser sensing unit 40 includes at least one area laser sensor. The laser sensing unit 40 can be fixed relative to the carrier 10, or can move on the carrier 10 along a set direction to achieve the scanning function in a certain range. The laser sensing unit 40 can scan the object 60 to be detected to determine a position of the odor sampling part 61 of the object 60 to be detected. Here, the object 60 to be detected can be various large or medium-and-small objects or areas, such as containers, trunks, parcels, vehicles and warehouses. The odor sampling part 61 is an area on the surface of the object 60 to be detected that communicates an internal odor space, such as a vent hole of a container, a zipper of a trunk or a transom of a warehouse.

In the present embodiments, the position of the odor sampling part of the object to be detected is determined by scanning the object to be detected through a laser sensing unit, and the electronic nose is adjusted to odor sampling part by an adjusting mechanism, so that the electronic nose can perform odor sampling at the odor sampling part, thereby meeting the relevant inspection requirements. Compared with the inspection process in the related art, the scanning of the laser sensing unit and adjustment of the adjusting mechanism can effectively simplify the inspection process of the electronic nose, reduce the time consumed by the inspection process, and improve the inspection efficiency.

Fig. 2 is a schematic structural view according to the electronic nose positioning device of the present disclosure. The electronic nose positioning device according to the present embodiment further includes a controller 50. The controller 50 can be configured to receive the scan data of the laser sensing unit 40 and cause the adjusting mechanism 30 to adjust the electronic nose 20 to the odor sampling part 61 of the object to be detected 60 based on the scan data.

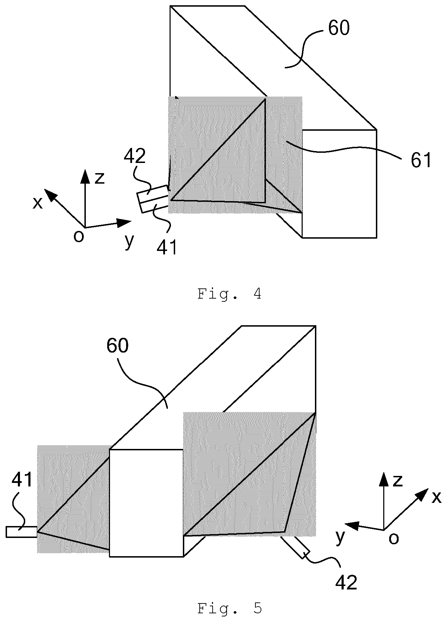

Referring to Fig. 4, the at least one area laser sensor includes a first area laser sensor 41 and a second area laser sensor 42. In Fig. 4, a rectangular coordinate system can be established with reference to a side surface of the object to be detected 60, wherein the direction parallel to the side surface of the object to be detected 60 and the horizontal plane is the x direction, the direction perpendicular to the side surface of the object to be detected 60 and parallel to the horizontal plane is the y direction, and the direction perpendicular to both the x and y directions is the z direction. In this way, the scanning surface of the first area laser sensor 41 is a vertical scanning surface parallel to the zy plane, which can be used to collect the side profile information of the object 60 to be detected. The scanning area of the second area laser sensor 42 is a horizontal scanning plane parallel to the xy plane, which can be used to collect the position and motion information of the object 60 to be detected relative to the laser sensing unit 40, for example, to obtain a real-time position of the object 60 to be detected, and accordingly further calculate a speed or acceleration of the laser sensing unit 40 or the carrier 10 fixedly provided with the laser sensing unit 40 relative to the object 60 to be detected.

The dynamic scanning process of the first area laser sensor 41 and the second area laser sensor 42 can be realized by movement of the carrier relative to the object 60 to be detected, and can be realized by movement of the first area laser sensor 41 and the second area laser sensor 42 on the carrier 10.

In Fig. 4, the first area laser sensor 41 and the second area laser sensor 42 are located on the same side of the object 60 to be detected. That is, the second area laser sensor 42 is located on one side of the object 60 to be detected adjacent to the first area laser sensor 41. In other embodiments, referring to Fig. 5, the second area laser sensor 42 can also be located on one side of the object 60 to be detected away from the first area laser sensor 41. Correspondingly, the carrier 10 can be a gantry frame moving along the track, which can move on both sides of and over the object 60 to be detected (for example, a container), and the first area laser sensor 41 and the second area laser sensor 42 can be respectively disposed on the frame bodies of the gantry frame located on both sides of the object 60 to be detected. Correspondingly, the electronic nose 20 and the adjusting mechanism 30 can be provided on the gantry frame.

The controller 50 receives scan data of the first area laser sensor 41 and the second area laser sensor 42 and establishes a side profile image of the object 60 to be detected according to the scan data. The controller 50 also calculates a position of the odor sampling part 61 of the object 60 to be detected in the side profile image according to the scan data, for example, determines the horizontal distance and vertical distance from the odor sampling part 61 to the edge of the side surface of the object 60 to be detected, and then causes the adjusting mechanism 30 to adjust the electronic nose 20 to the odor sampling part 61.

If the current position of the electronic nose 20 is far from the odor sampling part 61, the controller 50 can calculate a position of the odor sampling part 61 of the object 60 to be detected on the side profile image based on the scan data, so that the motion range of the carrier 10 moving to the distal end of the robot arm 31 can cover a position of the odor sampling part 61. In this way, the controller 50 can further cause the robot arm 31 to adjust the sampler of the electronic nose 20 to the odor sampling part 61, for example, to make the sampler directly face towards the odor sampling part 61.

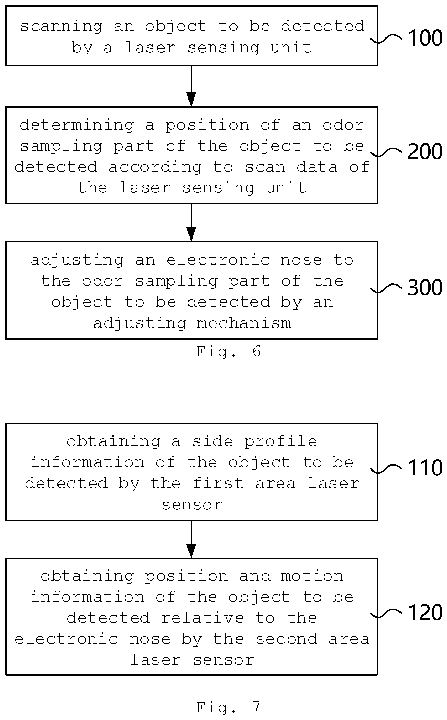

With reference to the above-described embodiments of the electronic nose positioning device, the present disclosure also provides a plurality of embodiments of the electronic nose positioning method. Fig. 6 is a schematic flow chart of the electronic nose positioning method of the present disclosure. In Fig. 6, the electronic nose positioning method includes the steps of:
step 100: scanning an object 60 to be detected by a laser sensing unit 40;
step 200: determining a position of an odor sampling part 61 of the object 60 to be detected according to scan data of the laser sensing unit 40.
step 300: adjusting an electronic nose 20 to the odor sampling part 61 of the object 60 to be detected by an adjusting mechanism 30.

Still taking as an example that the object to be detected 60 includes a container and the odor sampling part 61 is a vent hole of the container, when there is a need to inspect the container, it is possible to first scan the side surface of the container by the laser sensing unit 40, and determine the specific position of the vent hole of the container on the side surface. Then, the electronic nose can be adjusted to the vent hole according to the specific position, so that the electronic nose can collect the odor in the vent hole, thereby realizing the relevant inspection of the container.

In the above-described steps 100 to 300, the control of the laser sensing unit 40, the adjusting mechanism 30 and the electronic nose 20 can be implemented by the controller within the electronic nose positioning device, the remote controller of the operator or the remote-control platform.

Referring to Fig. 7, the laser sensing unit 40 comprises a first area laser sensor 41 whose scanning surface is a vertical scanning surface and a second area laser sensor 42 whose scanning surface is a horizontal scanning surface, and the step 100 includes steps 110 and 120. In step 110, the side profile information of the object 60 to be detected is collected by the first area laser sensor 41. In step 120, the position and motion information of the object to be detected 60 relative to the electronic nose 20 is collected by the second area laser sensor 42. The step 110 and the step 120 can be interchanged in sequence and can be performed simultaneously.

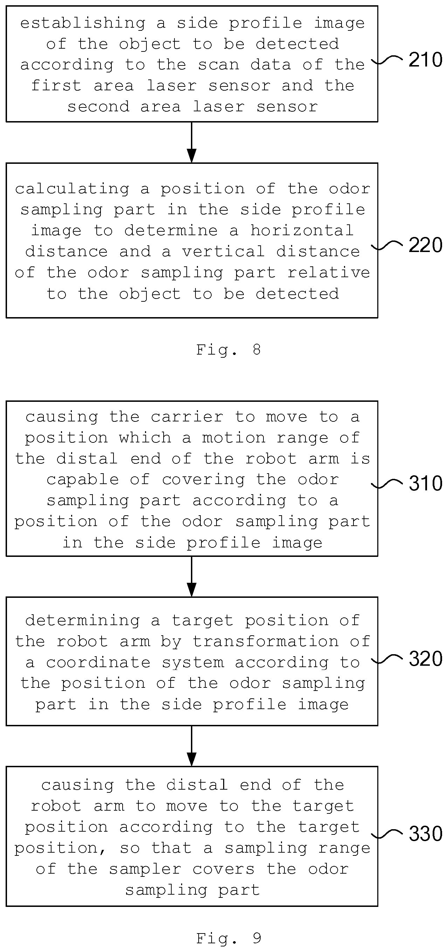

Referring to Fig. 8, the step 200 includes step 210 and step 220. In step 210, a side profile image of the object 60 to be detected is established according to the scan data of the first area laser sensor 41 and the second area laser sensor 42. In step 220, a position of the odor sampling part 61 in the side profile image is calculated to determine a horizontal distance and a vertical distance of the odor sampling part 61 relative to an edge of the object 60 to be detected.

For example, a rectangular profile image of the side surface of the container is established, and the edge position of the side surface of the container and the specific position of the vent hole on the side surface of the container can be clearly identified from the image. This position can be defined by the distance from the edge position of the side surface of the container. For example, the position of the vent hole is defined by a horizontal distance L between the vent hole and the edges on both sides in a vertical direction, as well as a vertical distance H between the vent hole and the bottom edge of the side surface of the container or the flat surface or ground where the container is placed.

In addition, the first area laser sensor 41 and the second area laser sensor 42 can also be used to determine the distance of the carrier 10 relative to the object 60 to be detected, so that the controller causes the carrier 10 to move to a position closer to the object 60 to be detected, thereby facilitating adjusting the sampling position of the electronic nose 20 by the adjusting mechanism 30.

In Fig. 3, the carrier 10 is movable. The electronic nose 20 includes a host 21 and a sampler 22. The host 21 is disposed on the carrier, and the adjusting mechanism 30 includes a robot arm 31, which is disposed on the carrier 10 or the host 21, wherein the sampler 22 is disposed at a distal end of the robot arm 31. On such basis, referring to Fig. 9, in some embodiments, the step 300 can include step 310 to step 330.

In step 310, according to the position of the odor sampling part 61 in the side profile image, the carrier is caused to move to a position that the motion range of the distal end of the robot arm 31 can cover the odor sampling part 61. In such step, there is just need for causing the carrier to move to the position that the motion range of the distal end of the robot arm 31 can cover the odor sampling part 61, and no need to make accurate movement of the carrier in place. Thus, it is equivalent to a fuzzy positioning process. A more accurate positioning process of the electronic nose sampler can be implemented by the steps 320 and 330.

Of course, if the carrier is in a short distance from the odor sampling part 61, even if the carrier does not move, the motion range of the distal end of the robot arm 31 can also cover the position of the odor sampling part 61, and thus the step 310 can be omitted.

In step 320, the target position of the robot arm 31 can be determined by transformation of the coordinate system according to the position of the odor sampling part 61 in the side profile image. For example, it is possible to set the o point of the foregoing coordinate system on the second area laser sensor 42, and determine the x-coordinate value and the z-coordinate value in the foregoing coordinate system according to the distances between the odor sampling part 61 and the side edge and between the odor sampling part 61 and the bottom surface measured by the second area laser sensor 42 and the first area laser sensor 41, thereby further determining the coordinate position of the odor sampling part 61 relative to the second area laser sensor 42. Then, according to a positional relationship between the second area laser sensor 42 and the robot arm 31, the coordinate position of the odor sampling part 61 relative to the second area laser sensor 42 is converted to the coordinate system of the robot arm 31, to determine the target position of a distal end of the robot arm 31.

After the target position of a distal end of the robot arm 31 is determined, in step 330, the distal end of the robot arm 31 can be caused to move to the target position according to the target position, so that the sampling range of the sampler 22 covers the odor sampling part 61. In this process, it can be determined whether the sampler of the electronic nose is in place according to the feedback information of the detection switch, and after it is determined to be in place, the gas sampling process is maintained for a preset time until the end of the sampling.

Hereto, various embodiments of the present disclosure have been described in detail. Some details well known in the art are not described to avoid obscuring the concept of the present disclosure. According to the above description, those skilled in the art would fully know how to implement the technical solutions disclosed herein.

## Claims

1. An electronic nose positioning device, comprising:
a carrier (10);
an electronic nose (20) disposed on the carrier (10);
an adjusting mechanism (30) configured to adjust the electronic nose (20) to an odor sampling part (61) of an object (60) to be detected;
wherein the electronic nose positioning device further comprises a laser sensing unit (40) disposed on the carrier (10), the electronic nose (20), or the adjusting mechanism (30), and configured to scan the object (60) to be detected, so as to determine a position of an odor sampling part (61) of the object (60) to be detected,
wherein the laser sensing unit (40) comprises at least one area laser sensor, and the at least one area laser sensor comprises:
a first area laser sensor (41), whose scanning surface is a vertical scanning surface, configured to obtain side profile information of the object (60) to be detected; and
a second area laser sensor (42), whose scanning surface is a horizontal scanning surface, configured to obtain position and motion information of the object (60) to be detected relative to the electronic nose (20);
wherein the electronic nose positioning device further comprises:
a controller (50) configured to receive scan data of the first area laser sensor (41) and the second area laser sensor (42) and establish a side profile image of the object (60) to be detected according to the scan data;
wherein the controller (50) is configured to calculate a position of the odor sampling part (61) in the side profile image to determine a horizontal distance and a vertical distance of the odor sampling part (61) relative to the object (60) to be detected, and to cause the adjusting mechanism (30) to adjust the electronic nose (20) to the odor sampling part (61).

2. The electronic nose positioning device according to claim 1, wherein the carrier (10) is movable.

3. The electronic nose positioning device according to claim 1, wherein the electronic nose (20) comprises a host (21) and a sampler (22), the adjusting mechanism (30) comprises: a robot arm (31) disposed on the carrier (10) or the host (21), and the sampler (22) is disposed at a distal end of the robot arm (31).

4. The electronic nose positioning device according to claim 1, wherein the carrier (10) is movable, the electronic nose (20) comprises a host (21) and a sampler (22), the adjusting mechanism (30) comprises: a robot arm (31) disposed on the carrier (10) or the host (21), and the sampler (22) is disposed at a distal end of the robot arm (31); and
wherein the controller (50) is configured to calculate a position of the odor sampling part (61) of the object (60) to be detected in the side profile image according to the scan data and cause the carrier (10) to move to a position which a motion range of the distal end of the robot arm (31) is capable of covering the odor sampling part (61).

5. The electronic nose positioning device according to claim 1, wherein the second area laser sensor (42) is configured to be located on one side of the object (60) to be detected adjacent to the first area laser sensor (41) or located on one side of the object (60) to be detected away from the first area laser sensor (41).

6. The electronic nose positioning device according to claim 1, wherein the adjusting mechanism (30) is configured to adjust the electronic nose (20) to a vent hole of a container, the laser sensing unit (40) is configured to scan the container, so as to determine a position of the vent hole of the container.

7. An electronic nose positioning method, comprising the steps of:
scanning an object (60) to be detected by a laser sensing unit (40) ;
determining a position of an odor sampling part (61) of the object (60) to be detected according to scan data of the laser sensing unit (40); and
adjusting an electronic nose (20) to the odor sampling part (61) of the object (60) to be detected by an adjusting mechanism (30), wherein the laser sensing unit (40) comprises a first area laser sensor (41) whose scanning surface is a vertical scanning surface and a second area laser sensor (42) whose scanning surface is a horizontal scanning surface, and the step of scanning the object (60) to be detected by the laser sensing unit (40) comprises:
obtaining a side profile information of the object (60) to be detected by the first area laser sensor (41); and
obtaining position and motion information of the object (60) to be detected relative to the electronic nose (20) by the second area laser sensor (42);
wherein the step of determining a position of an odor sampling part (61) of the object (60) to be detected comprises:
establishing a side profile image of the object (60) to be detected according to the scan data of the first area laser sensor (41) and the second area laser sensor (42);
calculating a position of the odor sampling part (61) in the side profile image to determine a horizontal distance and a vertical distance of the odor sampling part (61) relative to the object (60) to be detected; and
causing the adjusting mechanism (30) to adjust the electronic nose (20) to the odor sampling part (61).

8. The electronic nose positioning method according to claim 7, wherein the electronic nose (20) comprises a host (21) and a sampler (22), the host (21) is disposed on a movable carrier, the adjusting mechanism (30) comprises a robot arm (31) disposed on the carrier or the host (21), and the sampler (22) is disposed at a distal end of the robot arm (31);
the step of adjusting an electronic nose (20) comprises:
causing the carrier (10) to move to a position which a motion range of the distal end of the robot arm (31) is capable of covering the odor sampling part (61) according to a position of the odor sampling part (61) in the side profile image;
determining a target position of the robot arm (31) by transformation of a coordinate system according to the position of the odor sampling part (61) in the side profile image; and
causing the distal end of the robot arm (31) to move to the target position according to the target position, so that a sampling range of the sampler (22) covers the odor sampling part (61).

## Patentansprüche

1. Positionierungsvorrichtung für eine elektronische Nase, umfassend:
einen Träger (10);
eine elektronische Nase (20), die auf dem Träger (10) angeordnet ist;
einen Einstellmechanismus (30), der zum Einstellen der elektronischen Nase (20) auf einen Geruchsprobenahmeteil (61) eines zu erfassenden Gegenstands (60) ausgelegt ist;
wobei die Positionierungsvorrichtung für eine elektronische Nase ferner eine Laserabtasteinheit (40) umfasst, die auf dem Träger (10), der elektronischen Nase (20) oder dem Einstellmechanismus (30) angeordnet und ausgelegt ist, um den zu erfassenden Gegenstand (60) abzutasten, um so eine Position eines Geruchsprobenahmeteils (61) des zu erfassenden Gegenstands (60) zu bestimmen,
wobei die Laserabtasteinheit (40) mindestens einen Lasersensor für einen Bereich umfasst, und wobei der mindestens eine Lasersensor für einen Bereich Folgendes umfasst:
einen Lasersensor für einen ersten Bereich (41), dessen Abtastoberfläche eine vertikale Abtastoberfläche ist und der ausgelegt ist, um Seitenprofilinformationen des zu erfassenden Gegenstands (60) zu erhalten; und
einen Lasersensor für einen zweiten Bereich (42), dessen Abtastoberfläche eine horizontale Abtastoberfläche ist und der ausgelegt ist, um Positions- und
Bewegungsinformationen des zu erfassenden Gegenstands (60) bezogen auf die elektronische Nase (20) zu erhalten;
wobei die Positionierungsvorrichtung für eine elektronische Nase ferner umfasst:
eine Steuerung (50), die zum Empfangen von Abtastdaten des Lasersensors für einen ersten Bereich (41) und des Lasersensors für einen zweiten Bereich (42) und zum Erstellen eines Seitenprofilbild des zu erfassenden Gegenstands (60) gemäß den Abtastdaten ausgelegt ist;
wobei die Steuerung (50) ausgelegt ist, um eine Position des Geruchsprobenahmeteils (61) in dem Seitenprofilbild zu berechnen, um einen horizontalen Abstand und einen vertikalen Abstand des Geruchsprobenahmeteils (61) bezogen auf den zu erfassenden Gegenstand (60) zu bestimmen und zu bewirken, dass der Einstellmechanismus (30) die elektronische Nase (20) auf das Geruchsprobenahmeteil (61) einstellt.

2. Positionierungsvorrichtung für eine elektronische Nase nach Anspruch 1, wobei der Träger (10) beweglich ist.

3. Positionierungsvorrichtung für eine elektronische Nase nach Anspruch 1, wobei die elektronische Nase (20) einen Host (21) und einen Probenehmer (22) umfasst; wobei der Einstellmechanismus (30) einen Roboterarm (31) umfasst, der an dem Träger (10) oder dem Host (21) angeordnet ist, und der Probenehmer (22) an einem distalen Ende des Roboterarms (31) angeordnet ist.

4. Positionierungsvorrichtung für eine elektronische Nase nach Anspruch 1, wobei der Träger (10) beweglich ist, die elektronische Nase (20) einen Host (21) und einen Probenehmer (22) umfasst; wobei der Einstellmechanismus (30) einen Roboterarm (31) umfasst, der an dem Träger (10) oder dem Host (21) angeordnet ist, und der Probenehmer (22) an einem distalen Ende des Roboterarms (31) angeordnet ist; und
wobei die Steuerung (50) ausgelegt ist, um eine Position des Geruchsprobenahmeteils (61) des zu erfassenden Gegenstands (60) in dem Seitenprofilbild gemäß den Abtastdaten zu berechnen und zu bewirken, dass der Träger (10) sich in eine Position bewegt, in der ein Bewegungsbereich des distalen Endes des Roboterarms (31) den Geruchsprobenahmeteil (61) abdecken kann.

5. Positionierungsvorrichtung für eine elektronische Nase nach Anspruch 1, wobei der Lasersensor für einen zweiten Bereich (42) ausgelegt ist, um sich auf einer Seite des zu erfassenden Gegenstands (60) neben dem Lasersensor für einen ersten Bereich (41) zu befinden oder sich auf einer Seite des zu erfassenden Gegenstands (60), die vom Lasersensor für einen ersten Bereich (41) weg weist, zu befinden.

6. Positionierungsvorrichtung für eine elektronische Nase nach Anspruch 1, wobei der Einstellmechanismus (30) ausgelegt ist, um die elektronische Nase (20) auf ein Entlüftungsloch eines Containers einzustellen; die Laserabtasteinheit (40) ausgelegt ist, um den Container abzutasten, um die Position des Entlüftungslochs des Containers zu bestimmen.

7. Positionierungsverfahren für eine elektronische Nase, umfassend die folgenden Schritte:
Abtasten eines zu erfassenden Gegenstands (60) durch eine Laserabtasteinheit (40); Bestimmen einer Position eines Geruchsprobenahmeteils (61) des zu erfassenden Gegenstands (60) gemäß den Abtastdaten der Laserabtasteinheit (40); und
Einstellen einer elektronischen Nase (20) auf einen Geruchsprobenahmeteil (61) des zu erfassenden Gegenstands (60) durch einen Einstellmechanismus (30),
wobei
die Laserabtasteinheit (40) einen Lasersensor für einen ersten Bereich (41), dessen Abtastoberfläche eine vertikale Abtastoberfläche ist, und eine Lasersensor für einen zweiten Bereich (42), dessen Abtastoberfläche eine horizontale Abtastoberfläche ist, umfasst und wobei der Schritt des Abtastens des zu erfassenden Gegenstands (60) durch die Laserabtasteinheit (40) umfasst:
Erhalten von Seitenprofilinformationen des zu erfassenden Gegenstands (60) durch den Lasersensor für einen ersten Bereich (41); und
Erhalten von Positions- und Bewegungsinformationen des zu erfassenden Gegenstands (60) bezogen auf die elektronische Nase (20) durch den Lasersensor für einen zweiten Bereich (42);
wobei der Schritt des Bestimmens einer Position eines Geruchsprobenahmeteils (61) des zu erfassenden Gegenstands (60) umfasst:
Erstellen eines Seitenprofilbildes des zu erfassenden Gegenstands (60) gemäß den Abtastdaten des Lasersensors für einen ersten Bereich (41) und des Lasersensors für einen zweiten Bereich (42);
Berechnen einer Position des Geruchsprobenahmeteils (61) in dem Seitenprofilbild, um einen horizontalen Abstand und einen vertikalen Abstand des Geruchsprobenahmeteils (61) bezogen auf den zu erfassenden Gegenstand (60) zu bestimmen; und
Bewirken, dass der Einstellmechanismus (30) die elektronische Nase (20) auf den Geruchsprobenahmeteil (61) einstellt.

8. Positionierungsverfahren für eine elektronische Nase nach Anspruch 7, wobei die elektronische Nase (20) einen Host (21) und einen Probenehmer (22) umfasst; wobei der Host (21) an einem beweglichen Träger angeordnet ist; der Einstellmechanismus (30) einen Roboterarm (31) umfasst, der an dem Träger oder dem Host (21) angeordnet ist, und der Probenehmer (22) an einem distalen Ende des Roboterarms (31) angeordnet ist;
der Schritt des Einstellens einer elektronischen Nase (20) umfasst:
Bewirken, dass der Träger (10) sich in eine Position bewegt, in der ein Bewegungsbereich des distalen Endes des Roboterarms (31) den Geruchsprobenahmeteil (61) gemäß einer Position des Geruchsprobenentnahmeteils (61) in dem Seitenprofilbild abdecken kann;
Bestimmen einer Zielposition des Roboterarms (31) durch Transformation eines Koordinatensystems gemäß der Position des Geruchsprofilteils (61) in dem Seitenprofilbild; und
Bewirken, dass das distale Ende des Roboterarms (31) sich gemäß der Zielposition in die Zielposition zu bewegt, sodass ein Probenahmebereich des Probenehmers (22) den Geruchsprobenahmeteil (61) abdeckt.

## Revendications

1. Dispositif de positionnement de nez électronique, comprenant :
un support (10) ;
un nez électronique (20) disposé sur le support (10) ;
un mécanisme d'ajustement (30) configuré pour ajuster le nez électronique (20) par rapport à une partie d'échantillonnage d'odeur (61) d'un objet (60) à détecter ;
dans lequel le dispositif de positionnement de nez électronique comprend en outre une unité de détection laser (40) disposée sur le support (10), le nez électronique (20), ou le mécanisme d'ajustement (30), et configuré pour balayer l'objet (60) à détecter, de manière à déterminer une position d'une partie d'échantillonnage d'odeur (61) de l'objet (60) à détecter,
dans lequel l'unité de détection laser (40) comprend au moins un capteur laser de zone, et l'au moins un capteur laser de zone comprend :
un premier capteur laser de zone (41), dont la surface de balayage est une surface de balayage verticale, configurée pour obtenir des informations de profil latéral de l'objet (60) à détecter ; et
un deuxième capteur laser de zone (42), dont la surface de balayage est une surface de balayage horizontale, configurée pour obtenir des informations de position et de mouvement de l'objet (60) à détecter par rapport au nez électronique (20) ;
dans lequel le dispositif de positionnement de nez électronique comprend en outre :
un dispositif de commande (50) configuré pour recevoir des données de balayage du premier capteur laser de zone (41) et du deuxième capteur laser de zone (42) et établir une image de profil latéral de l'objet (60) à détecter en fonction des données de balayage ;
dans lequel le dispositif de commande (50) est configuré pour calculer une position de la partie d'échantillonnage d'odeur (61) dans l'image de profil latéral pour déterminer une distance horizontale et une distance verticale de la partie d'échantillonnage d'odeur (61) par rapport à l'objet (60) à détecter, et pour amener le mécanisme d'ajustement (30) à ajuster le nez électronique (20) par rapport à la partie d'échantillonnage d'odeur (61).

2. Dispositif de positionnement de nez électronique selon la revendication 1,
dans lequel le support (10) est mobile.

3. Dispositif de positionnement de nez électronique selon la revendication 1,
dans lequel le nez électronique (20) comprend un hôte (21) et un échantillonneur (22), le mécanisme d'ajustement (30) comprend : un bras robotisé (31) disposé sur le support (10) ou l'hôte (21), et l'échantillonneur (22) est disposé au niveau d'une extrémité distale du bras robotisé (31).

4. Dispositif de positionnement de nez électronique selon la revendication 1,
dans lequel le support (10) est mobile, le nez électronique (20) comprend un hôte (21) et un échantillonneur (22), le mécanisme d'ajustement (30) comprend : un bras robotisé (31) disposé sur le support (10) ou l'hôte (21), et l'échantillonneur (22) est disposé au niveau d'une extrémité distale du bras robotisé (31) ; et
dans lequel le dispositif de commande (50) est configuré pour calculer une position de la partie d'échantillonnage d'odeur (61) de l'objet (60) à détecter dans l'image de profil latéral en fonction des données de balayage et amener le support (10) à se déplacer vers une position dans laquelle une plage de mouvement de l'extrémité distale du bras robotisé (31) est capable de couvrir la partie d'échantillonnage d'odeur (61).

5. Dispositif de positionnement de nez électronique selon la revendication 1,
dans lequel le deuxième capteur laser de zone (42) est configuré pour être situé sur un côté de l'objet (60) à détecter de manière adjacente au premier capteur laser de zone (41) ou situé sur un côté de l'objet (60) à détecter à distance du premier capteur laser de zone (41).

6. Dispositif de positionnement de nez électronique selon la revendication 1,
dans lequel le mécanisme d'ajustement (30) est configuré pour ajuster le nez électronique (20) par rapport à un trou d'aération d'un contenant, l'unité de détection laser (40) est configurée pour balayer le contenant, de manière à déterminer une position du trou d'aération du contenant.

7. Procédé de positionnement de nez électronique, comprenant les étapes de :
balayage d'un objet (60) à détecter par une unité de détection laser (40) ;
détermination d'une position d'une partie d'échantillonnage d'odeur (61) de l'objet (60) à détecter en fonction des données de balayage de l'unité de détection laser (40) ; et
ajustement d'un nez électronique (20) par rapport à la partie d'échantillonnage d'odeur (61) de l'objet (60) à détecter par un mécanisme d'ajustement (30),
dans lequel l'unité de détection laser (40) comprend un premier capteur laser de zone (41) dont la surface de balayage est une surface de balayage verticale et un deuxième capteur laser de zone (42) dont la surface de balayage est une surface de balayage horizontale, et l'étape de balayage de l'objet (60) à détecter par l'unité de détection laser (40) comprend :
l'obtention d'une information de profil latéral de l'objet (60) à détecter par le premier capteur laser de zone (41) ; et
l'obtention d'informations de position et de mouvement de l'objet (60) à détecter par rapport au nez électronique (20) par le deuxième capteur laser de zone (42) ;
dans lequel l'étape de détermination d'une position d'une partie d'échantillonnage d'odeur (61) de l'objet (60) à détecter comprend :
l'établissement d'une image de profil latéral de l'objet (60) à détecter en fonction des données de balayage du premier capteur laser de zone (41) et du deuxième capteur laser de zone (42) ;
le calcul d'une position de la partie d'échantillonnage d'odeur (61) dans l'image de profil latéral pour déterminer une distance horizontale et une distance verticale de la partie d'échantillonnage d'odeur (61) par rapport à l'objet (60) à détecter ; et
le fait d'amener le mécanisme d'ajustement (30) à ajuster le nez électronique (20) par rapport à la partie d'échantillonnage d'odeur (61).

8. Procédé de positionnement de nez électronique selon la revendication 7, dans lequel le nez électronique (20) comprend un hôte (21) et un échantillonneur (22), l'hôte (21) est disposé sur un support mobile, le mécanisme d'ajustement (30) comprend un bras robotisé (31) disposé sur le support ou l'hôte (21), et l'échantillonneur (22) est disposé au niveau d'une extrémité distale du bras robotisé (31) ;
l'étape d'ajustement d'un nez électronique (20) comprend :
le fait d'amener le support (10) à se déplacer vers une position dans laquelle une plage de mouvement de l'extrémité distale du bras robotisé (31) est capable de couvrir la partie d'échantillonnage d'odeur (61) en fonction d'une position de la partie d'échantillonnage d'odeur (61) dans l'image de profil latéral ;
la détermination d'une position cible du bras robotisé (31) par transformation d'un système de coordonnées en fonction de la position de la partie d'échantillonnage d'odeur (61) dans l'image de profil latéral ; et
le fait d'amener l'extrémité distale du bras robotisé (31) à se déplacer vers la position cible en fonction de la position cible, de sorte qu'une plage d'échantillonnage de l'échantillonneur (22) couvre la partie d'échantillonnage d'odeur (61).
